# EUROPEAN PATENT APPLICATION

(11) **EP 1 645 253 A2**
(43) Date of publication of application: **12.04.2006**
(21) Application number: 05111757.0
(22) Date of filing: 05.11.2001
(51) Int. Cl.: A61F 7/00

(54) **Cryogenic chamber for systemic cryogenic treatment**

(62) Divisional of application: 01274669.9
(71) Applicant: Brojek, Wieslaw, PL-01-424 Warszawa (PL); Szmurlo, Wlodzimierz, PL-01-424 Warszawa (PL)
(72) Inventor: Brojek, Wieslaw, PL-01-424 Warszawa (PL); Szmurlo, Wlodzimierz, PL-01-424 Warszawa (PL)
(74) Representative: Czyz, Zbigniew

(57) **Abstract**

Cryogenic chamber comprising a treatment chamber, adaptations halls, a feeding system and a control system characterized in that it has a form of a depression.

## Description

Present invention relates to a cryogenic chamber comprising a treatment chamber, adaptations halls, a feeding system and a control system for running a cryogenic therapy treatment in particular, during the regeneration for running a cryogenic stimulation treatment.

The following definition has been used in the following description.

The term "cryogenic chamber" describes a structure comprising one or more adaptation hall(s), which allows patients reaching a treatment chamber, where patients stays from 30 seconds to 3 minutes, a feeding system that feeds a treatment chamber with a cold air in the cryogenic temperature from -110°C to -160°C as well as control, protection and other suitable systems.

The term "cryogenic treatment" describes a process during which the whole patient's organism is placed within the treatment chamber, which is a part of the cryogenic chamber, and is subjected to the cold air in the cryogenic temperature.

The term "depression" describes a concavity, where gases that are heavier than the surrounding gases can be collected.

Around the world there are produced and used cryogenic chambers in the typical configuration comprising a treatment chamber and one or more adaptation hall together with the cold air generating unit. Known cryogenic chambers are build as structures with solid walls with good thermal isolation from the surrounding environment. The floor of the treatment chamber and adaptation hall defines a single plane. Cryogenic chambers are build in different sizes, using different technology of building thermal isolated walls, they have different additional equipment, different control systems and they comprise wide range of different unit for cooling down the treatment chamber.

From the German patent publication DE 3441091 there is know a cryogenic chamber comprising two combined rooms with thermal isolated walls, those rooms are treatment chamber and adaptation hall. The floor of the treatment chamber and the floor of the adaptation hall lie in the same plane. The treatment chamber and adaptation hall both have windows that allows personnel to monitor patients. The above mentioned rooms are connected with each other using a thermal isolated door. Within the adaptation hall there is maintained a temperature of -60°C, within the adaptation hall patients adapts to the cryogenic temperature which they encounter in the treatment chamber. The adaptation hall collects also cold air that is "flowing out" of the treatment chamber while patients enter and leave the treatment chamber. Within the treatment chamber cooled down with use of the heat exchanger fed with liquid nitrogen the temperature is maintained at the level of -150°C.

In the German patent publication DE 19515287 the cryogenic chamber comprising a treatment chamber and two adaptation halls is disclosed. Feeding system comprises three cooling units that cool down three streams of air cooled down to the temperature respectively of 0 -30°C, -60 -80°C and -60 -100°C, cooling in this way three rooms. The floors of the treatment chamber and adaptation halls lie in the same plane. All three rooms are connected with thermal isolated doors that allow patients to migrate between rooms.

From the German patent publication DE 29615726 there is known a cryogenic chamber, where the lighting is provided by placing windows in the top part of the chamber and placing lamps on the outside of the windows.

The major drawback of the known solutions is significant weight and outflow of the cooled heavy air from the cryogenic chamber through the doors outside the chamber, when patients enter and leave the treatment chamber. A significant weight of the chamber leads to the high lost of the cooling medium during startup procedure, while the outflow of the cooled heavy air leads to the increased lost of the cooling medium during the treatment.

The aim of the present invention is providing the cryogenic chamber for cryogenic treatment the whole body of the patient or patients, which possess the advantages of the known solutions, and did not posses drawbacks of the known solutions. The cryogenic chamber according to the invention should have simple and compact construction, and should comply with all safety requirements for the patients and servicing personnel. The investment costs and exploitation costs should be relatively small.

The above mentioned aim has been achieved according to the invention by creating a cryogenic chamber in the form of depression.

The construction block of the depression is divided into two functional parts, which are separated from the surrounding environment by the thermal isolated walls, the adaptation hall forms the first part, which floor constitutes a transport ramp in a form of stairs, that leads towards the depression that is placed lower, and the treatment chamber forms a second part, which is provided from the side of the transport ramp with the entrance opening.

Preferably the entrance opening to the adaptation hall is placed on the level of lower part of the depression and is constituted by the transport ramp in the form of stairs that runs upward up to the level of the horizontal deck that connects both transport ramps at the upper level of the depression.

The cryogenic chamber for the cryogenic treatment of the whole body of the patient or patients, according to the invention combines advantages of the know solutions and do not possess drawbacks of prior art solutions. Producing a cryogenic chamber in the form of a depression brings a number of advantages since there is no need of using external doors of dedicated construction with good thermal isolation properties, moreover patients adapt smoothly to the low temperature conditions, entering and leaving the cryogenic chamber is realized without any loss of the flowing out cold air. Making a treatment chamber, from a certain level, of transparent walls and providing a transparent roof together with placing a transparent cover between the treatment chamber and open from the top adaptation hall eliminates a filling of being enclosed to the person suffering of claustrophobia. The phenomena of deposition of cold air in the natural depressions is know from centuries, this knowledge led to the construction of the cellar or larder placed in the pits. The other analogy that presents the basic phenomenon is swimming basin which is entered from the top and not from the side, simply to avoid disadvantageous outflow of water.

The cryogenic chamber according the invention is entered from the top via the transport way, preferably, the upper part of the depression is placed at the floor level of the treatment room.

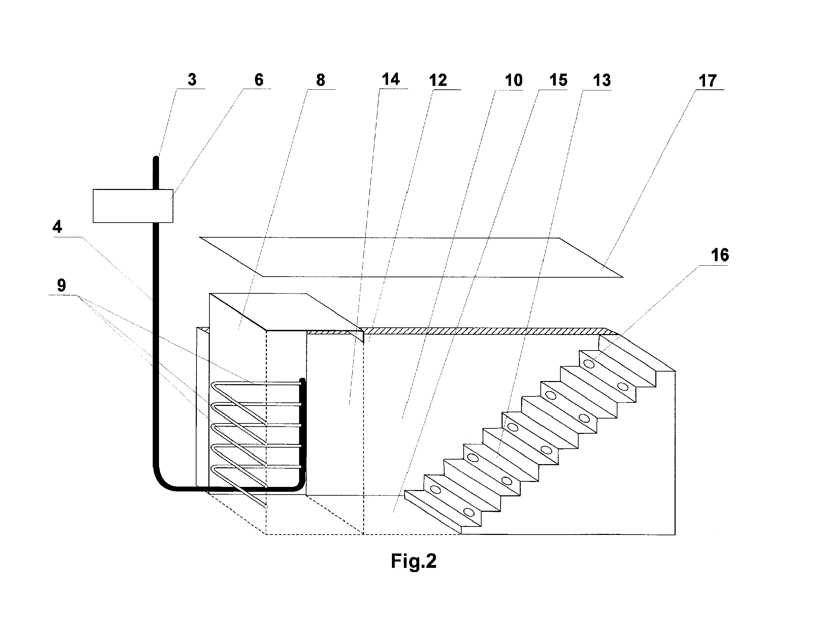

The present invention has been presented in the preferred embodiment on the drawing, where: fig. 1 shows in a cross-section the cryogenic chamber for using with the method according to the invention, fig. 2 shows in a perspective a cross-section of cryogenic chamber according to the invention, fig. 3 shows in the perspective view another embodiment of the cryogenic chamber according to the invention.

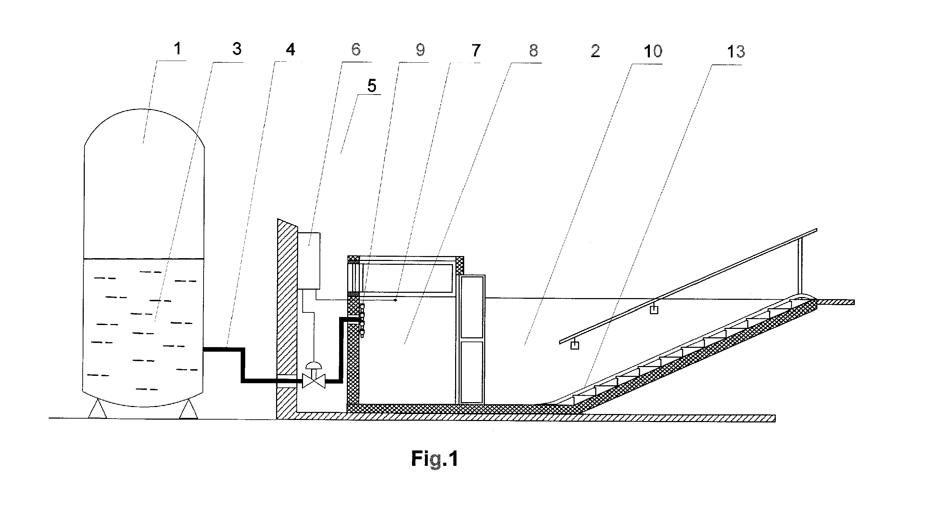

In the cryogenic chamber presented in the fig. 1 there is used a natural phenomenon of a heavier weight of cold air in relation to the weight of the air in the room temperature. Cryogenic chamber is in a form of concavity (depression) which is approached from the above. Its construction block is divided into two functional parts; first part is separated from the surrounding by the thermal isolated walls 12. Walls 12 defines adaptation hall 10, provided with the transport ramp 13. The transport ramp 13 leads down towards the treatment chamber 8. The treatment chamber 8 is placed in the lower part of the depression, it is provided with the entrance opening 14. Between the transport ramp 13 and treatment chamber 8 there is provided a flat part 15 of the adaptation hall. The cryogenic chamber 2 is provided with the lighting points 16 placed in the area of the transport ramp 13 of the adaptation hall 10, in the area of the flat part 15 of the adaptation hall 10 and within the treatment chamber 8. The upper part of the chamber is provided with the transparent cover 17. The parts of the installation that contain mediums in the cryogenic temperature are provided with thermal isolation that precludes direct contact of these elements with the patient's body.

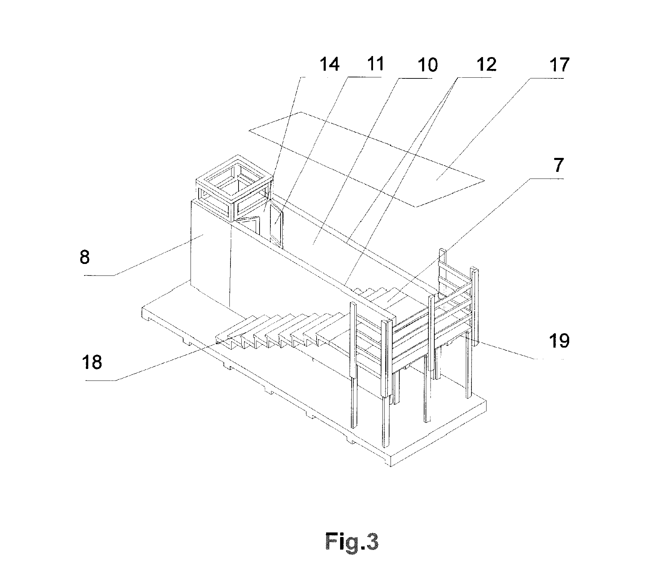

In the cryogenic chamber shown in the fig. 3 the entrance to the cryogenic chamber is placed at the level of the lower depression, it is defined by the transport ramp 18 leading upwards up to the horizontal deck 19 that connects both transport ramps at the upper level of the depression.

In another the embodiment of the cryogenic chamber (not shown) the treatment chamber is provided with a door that constitute an emergency exit.

## Claims

1. Cryogenic chamber comprising a treatment chamber (8), adaptations hall (10), feeding system and control system (6)
**characterized in that** it has a form of a depression.

2. Cryogenic chamber according to the claim 1
**characterized in that** its construction block is divided into two functional parts, which are separated from the surrounding by the thermal isolated walls (12), the adaptation hall (10) forms the first part, which floor constitutes a transport ramp (13) in a form of stairs, that leads towards the depression that is placed lower, and the treatment chamber (8) forms a second part, which is provided from the side of the transport ramp (13) with the entrance opening (14).

3. Cryogenic chamber according to the claim 1
**characterized in that** the entrance opening to the adaptation hall is placed on the level of lower part of the depression and is constituted by the transport ramp (18) in the form of stairs that runs upward up to the level of the horizontal deck (19) that connects both transport ramps at the upper level of the depression.
